# EUROPÄISCHE PATENTSCHRIFT

(11) **EP 1 812 553 B1**
(45) Veröffentlichungstag und Bekanntmachung des Hinweises auf die Patenterteilung: **04.02.2009**
(21) Anmeldenummer: 05779612.0
(22) Anmeldetag: 19.08.2005
(51) Int. Cl.: C12N 1/21, C12P 13/08

(54) **VERFAHREN ZUR FERMENTATIVEN HERSTELLUNG VON L-VALIN UNTER VERWENDUNG CORYNEFORMER BAKTERIEN MIT ERHÖHTER TRANSAMINASE C AKTIVITÄT**
PROCESS FOR THE FERMENTATIVE PRODUCTION OF L-VALINE EMPLOYING CORYNEFORME BACTERIA HAVING AN ENHANCED TRANSAMINASE C ACTIVITY
PROCÉDÉ DE PRODUCTION DE L-VALINE PAR FERMENTATION EN UTILISANT DES BACTÉRIES CORYNEFORMES À ACTIVITÉ DE TRANSAMINASE C RENFORCÉE

(30) Priorität: 28.09.2004 DE 102004046933
(43) Veröffentlichungstag der Anmeldung: 01.08.2007
(73) Patentinhaber: Forschungszentrum Jülich GmbH, 52425 Jülich (DE)
(72) Erfinder: MARIENHAGEN, Jan, 52428 Jülich (DE); EGGELING, Lothar, 52428 Jülich (DE); SAHM, Hermann, 52428 Jülich (DE)
(86) Internationale Anmeldenummer: PCT/DE2005/001465
(87) Internationale Veröffentlichungsnummer: WO 2006/034667

(56) Entgegenhaltungen:
- EP-A- 1 275 729
- EP-B- 1 155 139
- WANG, M.D. ET AL.: "Cloning of Genes That Suppress an Escherichia coli K-12 Alanine Auxotroph When Present in Multicopy Plasmids" JOURNAL OF BACTERIOLOGY, Bd. 169, Nr. 12, Dezember 1987 (1987-12), Seiten 5610-5614, XP002380800
- LEYVAL, D. ET AL.: "Characterisation of the enzyme activities involved in the valine biosynthetic pathway in a valine-producing strain of Corynebacterium glutamicum" JOURNAL OF BIOTECHNOLOGY, Bd. 104, Nr. 1-3, 2003, Seiten 241-252, XP002380799 in der Anmeldung erwähnt
- DATABASE NCBI [Online] 5. August 2004 (2004-08-05), NAKAGAWA, S.: "Complete genomic sequence of Corynebacterium glutamicum ATCC 13032" XP002380803 Database accession no. NC_003450 in der Anmeldung erwähnt
- MARIENHAGEN, J. ET AL.: "Functional Analysis of All Aminotransferase Proteins Inferred from the Genome Sequence of Corynebacterium glutamicum" JOURNAL OF BACTERIOLOGY, Bd. 187, Nr. 22, November 2005 (2005-11), Seiten 7639-7646, XP002380801

## Beschreibung

Die Erfindung betrifft ein Verfahren zur Herstellung von L-Valin sowie einen dafür geeigneten Mikroorganismus.

Die Aminosäure L-Valin, findet in der Humanmedizin, in der pharmazeutischen Industrie, der Lebensmittelindustrie und in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff oder die Zusammensetzung der Nährmedien wie z.B. die Zuckerkonzentration während der Fermentation oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und L- Aminosäuren produzieren. So ist zum Beispiel in US Patent 5,521,074 ein Corynebacterium Stamm beschrieben, der resistent gegenüber L-Valin ist und sensitiv gegenüber Fluoropyruvat. Ferner ist in EP 0287123 beschrieben, dass Corynebacterien mit Resistenz gegenüber Mycophenolsäuren vorteilhaft zur L-Valin Gewinnung benutzt werden können. Auch ist aus der EP 0519113 A1 und der US 5,658,766 bekannt, dass Mutanten mit mutierter Valyl-tRNA Synthetase in Kombination mit weiteren Mutationen für die L-Valinbildung herangezogen werden können. Daneben ist in der WO 001996006926 A1 ein Verfahren zur L-Valin Herstellung beschrieben, bei dem ein Mikroorganismus benutzt wird, der das Vitamin Liponsäure für das Wachstum benötigt und einen Defekt in der ATPase besitzt.

Die rekombinante DNA-Technik wird zusätzlich zur Verbesserung der intrinsischen Eigenschaften von L-Aminosäure produzierenden Stämmen von Corynebacterium eingesetzt. So ist in den Schriften EP 1155139B1 und EP 0356739 B1 beschrieben, dass Verstärkung der Expression der Biosynthesegene ilvBN, ilvC, ilvD vorteilhaft zur L-Valinbildung eingesetzt werden. Bekannt ist ferner aus der EP 1155139 B1, dass die Abschwächung oder Ausschaltung des Threonin-Dehydratase Gens ilvA und/oder von Genen der Pantothenatsynthese zur L-Valinbildung herangezogen werden können.

D. Leyval et al. offenbaren in dem Artikel "Characterisation of the enzyme activities involved in the valine biosynthetic pathway in a valine-producing strain of Corynebacterium glutamicum" im Journal of Biotechnology 104 (2003) 241 - 252 Elsevier eine kinetische Studie zum L-Valin Biosyntheseweg in Corynebacterium glutamicum bei der gezeigt wird, dass Ketoisovalerat mittels einer Transaminase C zu L-Valin umgesetzt wird.

Aus der EP 1 275 729 A1 geht hervor, dass die Erhöhung der Transaminase C-Aktivität zur Verringerung der L-Valin-Produktion führen kann.

Es ist die Aufgabe der Erfindung, ein Verfahren und einen dafür geeigneten Mikroorganismus zur verbesserten fermentativen Herstellung von L-Valin bereitzustellen.

Ausgehend vom Oberbegriff des Anspruchs 1 wird die Aufgabe erfindungsgemäß gelöst mit den im kennzeichnenden Teil des Anspruchs 1 angegebenen Merkmalen.

Es wurde gefunden, dass coryneforme Bakterien nach Verstärkung des für die Transaminase C kodierenden Gens in verbesserter Weise L-Valin produzieren. Mit dem erfindungsgemäßen Verfahren ist es nunmehr möglich, L-Valin mit einer Ausbeute herzustellen, die 35,8% höher liegt als die des nicht erfindungsgemäß veränderten Stammes.

In Folgenden soll die Erfindung beschrieben werden.

Gegenstand der Erfindung ist ein Verfahren zur fermentativen Herstellung von L-Valin unter Verwendung von coryneformen Bakterien, in denen die Aktivität der Transaminase C erhöht wird.

Bevorzugte Ausführungsformen finden sich in den Unteransprüchen.

Erfindungsgemäß wird die Aktivität der Transaminase C in einem, die Aminosäure L-Valin produzierenden coryneformen Bakterium verstärkt.

Die eingesetzten Stämme produzieren vorzugsweise bereits vor der Verstärkung des Transaminase-C-Gens L-Valin.

Als Mikroorganismus wird ein coryneformes Bakterium eingesetzt.

Besonders bevorzugt sind dabei Corynebacterium glutamicum, Corynebacterium acetoglutamicum, Corynebacterium thermoanimogenes, Brevibacterium flavum, Brevibacterium lactofermentum, Brevibacterium divaricatum.

Besonders geeignete Stämme der Gattung Corynebacterium, insbesondere der Art Corynebacterium glutamicum, sind beispielsweise die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte, L-Aminosäuren überproduzierende Mutanten bzw. Stämme.

Die coryneformen Bakterien die Gegenstand der vorliegenden Erfindung sind, können L-Valin beispielsweise aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum zu nennen, die in der Fachwelt für ihre Fähigkeit bekannt ist, L-Aminosäuren zu produzieren.

Unter dem Begriff "Verstärkung" werden im Sinne der Erfindung die Erhöhung der intrazellulären Aktivität der Transaminase C durch beispielsweise folgende Maßnahmen verstanden:
Erhöhung der Genexpression durch mindestens eine Maßnahme aus der Gruppe bestehend aus:
   - Veränderung der Signalstrukturen der Genexpression, wie beispielsweise durch Veränderung der
      Repressorgene,
      Aktivatorgene,
      Operatoren,
      Promotoren;
      Attenuatoren,
      Ribosomenbindungsstellen,
      des Startkodons,
      Terminatoren.
   - Einführen eines stärkeren Promoters, wie beispielsweise tac-Promotor oder einen IPTG-induzierbaren Promotor.
   - Erhöhen der Genkopienzahl, beispielsweise durch
      Einführen von Vektoren, wie Plasmide,
      Erhöhung der endogenen Genkopienzahl, also das Einbringen von weiteren für die Transaminase C kodierenden Genen oder Allelen davon in das chromosomale Genom.
Weiterhin kann eine Verstärkung der Transaminase C Aktivität durch folgende Maßnahmen bewirkt werden:
   - Erhöhung der m-RNA - Stabiltät, beispielsweise durch Mutation der terminalen Positionen, welche den Abbruch der Transskription steuern.
Beispielsweise kann durch Stabilität der m-RNA des Transaminase C Gens eine verbesserte Produktbildung erreicht werden, indem die Stabilität durch zusätzliche und/oder veränderte Sequenzen am 5'-Ende oder 3'-Ende des Gens positiv beeinflusst wird. Allgemeine Beispiele dazu sind für Gene aus Bacillus subtilis (Microbiology 2001, 147:1331-41) oder Hefe (Trends Biotechnol. 1994, 12:444-9).
   - Verwendung eines Gens oder Allels, welches für ein entsprechendes Enzym einer erhöhten Aktivität kodiert.

Die Erhöhung der intrazellulären Aktivität eines oder mehrerer Enzyme (Proteine) in einem Mikroorganismus, die durch die entsprechende DNA kodiert werden, kann verstärkt werden, indem man beispielsweise einen starken Promotor oder ein Gen bzw. Allel verwendet, das für ein entsprechendes Enzym mit einer erhöhten Aktivität kodiert bzw. das entsprechende Gen (Protein) überexprimiert und gegebenenfalls diese Maßnahmen kombiniert.

Das Einführen eines stärkeren Promoters, wie z. B. den tac-Promoter (Amann et al (Gene 1988 69:301-15), oder Promotoren aus der Gruppe der bei Patek et al (Microbiology 1996 142:1297) beschriebenen Promotoren ist bevorzugt. Beispiele finden sich in der WO 96/15246 oder in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pätek et al. (Microbiology 142: 1297 (1996)), Knippers ("Molekulare Genetik", 6th edition, Georg Thieme Verlag, Stuttgart, Germany, 1995) oder auch bei Winnacker ("Gene und Klone", VCH Verlagsgesellschaft, Weinheim, Germany, 1990).

Die natürliche Nukleotidsequenz des Transaminase-C-Gens ist zwangsläufig durch die Erstellung der kompletten Genomsequenz von C. glutamicum bekannt (Kalinowski et al., 2003, J Biotechnol., 104:5-25; Ikeda M, and Nakagawa S. 2003 Appl. Microbiol. Biotechnol. 62:99-109), ohne das aber die Zuordnung eines offenen Leserahmens zur Transaminase C bekannt ist. Es ist auch bekannt, dass C. glutamicum (Leyval et al. 2003. J. Biotechnol.104:241-52) wie auch E. coli z. B. eine Transaminase C Aktivität besitzt (Wang et al. 1987. J. Bacteriol. 169:4228-4234). Das wie nachfolgend im Beispiel beschriebene und identifizierte offene Leseraster, das für Transaminase C kodiert, trägt die Nummer NCg12510 und ist in der öffentlich zugänglichen Datenbank des "National Institutes of Health" hinterlegt (http://www.ncbi.nlm.nih.gov), das identische Gen ist auch unter Cg12599 in der öffentlich zugänglichen "DNA Data bank of Japan" (http://gib.genes.nig.ac.jp) identifiziert.

Das unter diesen Nummern beschriebene Transaminase-C-Gen wird erfindungsgemäß vorzugsweise eingesetzt. Weiterhin können Allele des Transaminase-C-Gens verwendet werden, die sich beispielsweise aus der Degeneriertheit des genetischen Codes oder durch funktionsneutrale Sinnmutationen (sense mutations) oder durch Deletion oder Insertion von Nukleotiden ergeben.

Zur Erzielung einer Verstärkung kann entweder die Expression des Transaminase-C-Gens oder die katalytischen Eigenschaften des Enzymproteins heraufgesetzt bzw. verstärkt werden. Ebenfalls kann die katalytische Eigenschaft des Enzymproteins hinsichtlich ihrer Substratspezifität verändert werden. Gegebenenfalls können beide Maßnahmen kombiniert werden.

Die Verstärkung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (J. Bacteriol. 1988. 170: 5949), bei Voskuil und Chambliss (Nucleic Acids Res. 1998. 26: 3548, bei Jensen und Hammer (Biotechnol. Bioeng. 1998 58: 191), bei Patek et al. (Microbiology 1996. 142: 1297 und in bekannten Lehrbüchern der Genetik und Molekularbiologie wie z. B. dem Lehrbuch von Knippers ("Molekulare Genetik", 8. Auflage, Georg Thieme Verlag, Stuttgart, Deutschland, 2001) oder dem von Winnacker ("Gene und Klone", VCH Verlagsgesellschaft, Weinheim, Deutschland, 1990).

Mutationen, die zu einer Veränderung der katalytischen Eigenschaften von Enzymproteinen führen, insbesondere zu einer veränderten Substratspezifität, sind aus dem Stand der Technik bekannt. Als Beispiele seien die Arbeiten von Yano et al. 1998 Proc Natl Acad Sci U S A. 95:5511-5, Oue S. et al. J Biol Chem. 1999, 274:2344-9. und Onuffer et al. Protein Sci. 1995 4:1750-7 genannt, in denen die Veränderung der Spezifität von Aspartataminotransferasen offenbart ist. Als Mutationen kommen Transitionen, Transversionen, Insertionen und Deletionen in Betracht, sowie Methoden der gerichteten Evolution. Anleitungen zur Erzeugung derartiger Mutationen und Proteine gehören zum Stand der Technik und können bekannten Lehrbüchern (R. Knippers "Molekulare Genetik", 8. Auflage, 2001, Georg Thieme Verlag, Stuttgart, Deutschland), oder Übersichtsartikeln (N. Pokala 2001, J. Struct. Biol. 134:269-81; A. Tramontano 2004, Angew. Chem. Int. Ed Engl. 43:3222-3; N. V. Dokholyan 2004, Proteins. 54:622-8; J. Pei 2003, Proc. Natl. Acad. Sci. U S A. 100:11361-6; H. Lilie 2003, EMBO Rep. 4:346-51; R. Jaenicke Angew. Chem. Int. Ed. Engl. 42:140-2) entnommen werden.

Die Expression der Gene oder der mutierten Gene erfolgt vorzugsweise nach gebräuchlichen Methoden der Erhöhung der Kopienzahl durch Einbau in geeignete Plasmide. Als Plasmide eignen sich solche, die in coryneformen Bakterien repliziert werden. Zahlreiche bekannte Plasmidvektoren wie z. B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden (O. Kirchner 2003, J. Biotechnol. 104:287-99). Ebenso können Vektoren mit regulierbarer Expression benutzt werden, wie zum Beispiel pEKEx2 (B. Eikmanns, 1991 Gene 102:93-8; O. Kirchner 2003, J. Biotechnol. 104:287-99). Auch kann das Gen durch Integration in das Chromosom in einfacher Kopie (P. Vasicova 1999, J. Bacteriol. 181:6188-91), oder mehrfacher Kopie (D. Reinscheid 1994 Appl. Environ Microbiol 60:126-132) exprimiert werden.

Die Transformation des gewünschten Stammes mit dem Vektor zur Erhöhung der Kopienzahl erfolgt durch Konjugation oder Elektroporation des gewünschten Stammes von beispielsweise C. glutamicum. Die Methode der Konjugation ist beispielsweise bei Schäfer et al. (Applied and Environmental Microbiology (1994) 60:756-759) beschrieben. Methoden zur Transformation sind beispielsweise bei Tauch et al. (FEMS Microbiological Letters (1994)123:343-347) beschrieben.

Auf diese Weise kann das Transaminase-C-Gen oder dessen Allel in C. glutamicum exprimiert und überexprimiert werden.

Weiterhin kann es für die Produktion von L-Valin vorteilhaft sein, zusätzlich zur Erhöhung der Transaminase-C-Aktivität eines oder mehrere der Gene ausgewählt aus der Gruppe
- die für die Acetohydroxysäuresynthase kodierenden ilvBN-Gene,
- das für die Isomeroreduktase kodierende ilvC-Gen,
- das für die Dehydratase kodierende ilvD-Gen,
zu verstärken, insbesondere zu überexprimieren oder Allele dieser Gene, insbesondere
- die für feedback-resistente Acetohydroxysäuresynthase kodierenden ilvBN-Gene,
zu verstärken oder zu überexprimieren, um die Produktion von L-Valin weiter zu steigern.
Weiterhin kann es für die Produktion von L-Valin vorteilhaft sein, zusätzlich zur Erhöhung der Transaminase-C-Aktivität eines oder mehrere der Gene ausgewählt aus der Gruppe
- die für die Pantothenatsynthese kodierenden panBCD-Gene,
- die für die Liponsäuresynthese kodierenden lipAB-Gene,
- die für die Pyruvatdehydrogenase kodierenden aceE-, a-ceF, IpD-Gene,
- die für die Gene der ATP synthase A Untereinheit, ATP synthase B Untereinheit, ATP synthase C Untereinheit, ATP synthase alpha Untereinheit, ATP synthase gamma Untereinheit, ATP synthase Untereinheit, ATP synthase epsilon Untereinheit, ATP synthase delta Untereinheit
zu inaktivieren oder in ihrer Expression zu reduzieren oder zu mutieren, um funktionell abgeschwächte Genprodukte zu bilden, um die Produktion von L-Valin zu steigern.

Die erfindungsgemäß hergestellten coryneformen Bakterien können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Valin-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Mikroorganismen genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden.
Diese Stoffe können einzeln oder als Mischung verwendet werden.

Als Stickstoffquelle können organische, Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden.
Die Stickstoffquellen können einzeln oder als Mischung verwendet werden.

Als Phosphorquelle können Kaliumdihydrogenphosphat oder Dikaliumhydrogenphosphat oder die entsprechenden Natriumhaltigen Salze verwendet werden.

Das Kulturmedium soll weiterhin Salze von Metallen enthalten wie z.B. Magnesiumsulfat oder Eisensulfat, die für das Wachstum notwendig sind. Schließlich können essentielle Wuchsstoffe, wie Aminosäuren und Vitamine zusätzlich zu den oben genannten Stoffen eingesetzt werden.
Die genannten Einsatzstoffe können zur Kultur in Form eines einmaligen Ansatzes hinzu gegeben oder in geeigneter Weise während der Kultivierung zugefüttert werden.

Zur pH - Kontrolle der Kultur können basische Verbindungen wie Natriumhydroxid, Kaliumhydroxid, Ammoniak oder saure Verbindungen wie Phosphorsäure oder Schwefelsäure in geeigneter Weise eingesetzt werden. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe, z. B. Antibiotika, hinzugefügt werden. Um aerobe Bedingungen aufrechtzuerhalten werden Sauerstoff oder Sauerstoff-haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an L-Valin gebildet hat. Dieses Ziel wird normalerweise innerhalb von 10 Stunden bis 160 Stunden erreicht.

### Beispiele

### Beispiel 1: Klonierung der Transaminase C

Mit Hilfe der PCR Reaktion wurde ein DNA-Fragment, das das Transaminase-C-Gen enthält, amplifiziert. Es wurden die folgenden Primer benutzt:
orf2841-for:
   5'-ATGGTA(GGTCT)CAAATGTCTCTTATGAAGCCAAGCACTAG-3
orf2841-rev:
   5'-ATGGTA(GGTCT)CAGCGCTTTTTTTGATGAATTCTCCGATTTTG-3'

Die angegebenen Primer wurden durch MWG Biotech synthetisiert, und die PCR Reaktion entsprechend Standard Protokollen durchgeführt (Innis et al. PCR Protocols. A guide to Methods and Applications. 1990. Academic Press). Mit den Primern wurde ein DNA-Fragment von etwa 1,1 kb amplifiziert, das für Transaminase C kodiert. Die Primer enthalten zusätzlich die Schnittstelle des Restriktionsenzyms BsaI, die in obigen Nukleotidsequenzen in Klammer angegeben sind.

Das amplifizierte DNA-Fragment von etwa 1,1 kb wurde im 0.8%igen Agarosegel identifiziert und aus dem Gel mit bestehenden Methoden isoliert (QIAquik Gel Extraction Kit, Quiagen, Hilden). Die Ligation des Fragments erfolgte mit dem SureCloning Kit (Amersham, UK) in den Expressionsvektor pASK-IBA-3C (IBA, Göttingen). Mit dem Ligationsansatz wurde E. coli DH5 transformiert (Grant et al., 1990. Proceedings of the National of Sciences of the United States of America USA, 87:4645-4649). Die Selektion Plasmid enthaltender Stämme erfolgte durch Ausplattieren des Transformationsansatzes auf 25 mg pro Liter Chloramphenicol enthaltenden LB Platten.

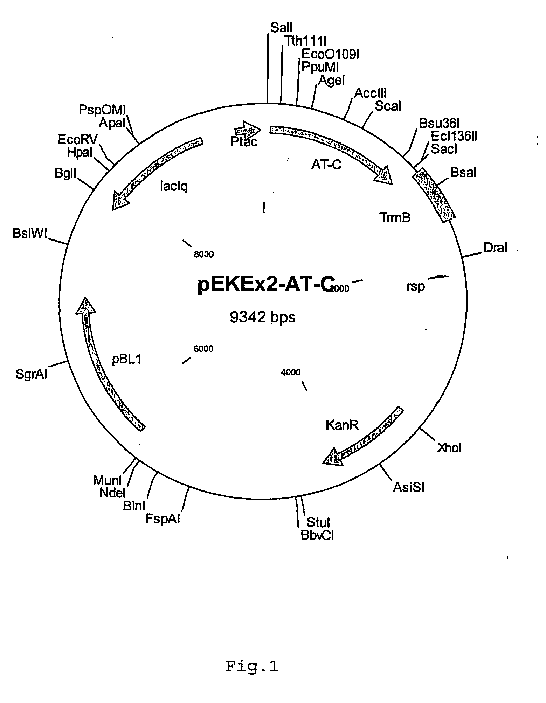

Nach Plasmidisolation wurden erhaltene Plasmide durch Restriktionsverdau und gelelektrophoretische Analyse charakterisiert. Das erhaltene Plasmid wurde als pASK-IBA-3Corf2841 bezeichnet. Es ist in Figur 1 angegeben.

### Beispiel 2: Isolierung der Transaminase C

E. coli DH5 mit pASK-IBA-3Corf2841 wurde bei 30°C in 100 ml LB mit 25 mg pro Liter Chloramphenicol bis zu einer optischen Dichte von 0,5 angezogen. Dann wurden 0,01 ml einer Anhydrotetracyclinlösung zugefügt, die 2 mg Anhydrotetracyclin pro Milliliter Dimethylformamid enthielt. Die Kultur wurde weiter für 3 Stunden bei 30 °C inkubiert. Anschließend wurden die Zellen 12 Minuten bei 4°C und 5000 Umdrehungen pro Minute durch Zentrifugation geerntet. Danach wurde das Zellpellet in Waschpuffer (100 mM Trihydroxymethylaminomethan, 1 mM Äthylenediaminetetraessigsäure, pH 8) resuspendiert und in ein Eppendorf-Reaktionsgefäß überführt. Der Zellaufschluss erfolgte bei 0 °C durch einen Ultraschalldesintegrator (Branson Sonifier W-250, Branson Sonic Power Company, Danbury, USA; Beschalldauer 10 min, Pulslänge 20 %, Beschallintensität 2). Nach der Ultraschallbehandlung wurden die Zelltrümmer durch Zentrifugation (30 min, 13000 Upm, 4 °C) abgetrennt und Rohextrakt als Überstand erhalten.

Zur Isolierung des Proteins wurden StrepTactin-Affinitätssäulen des Herstellers IBA (IBA, Göttingen, Deutschland) mit 1 ml Bettvolumen StrepTactin-Sepharose befüllt. Nach Äquilibrierung der Säulen mit Waschpuffer des Herstellers IBA wurde 1 ml des Rohextraktes auf die Sepharose gegeben. Nach Durchlauf des Extraktes wurde die Affinitätssäule fünfmal mit 1 ml Waschpuffer gewaschen. Die Elution des Transaminase-C-Proteins wurde mit Elutionspuffer, bestehend aus 100 mM Tris, 1 mM EDTA, 2,5 mM Desthiobiotin, pH 8, durchgeführt. Die Elutionsfraktionen wurden aliquotiert, bei -20 °C eingefroren, und direkt im Enzymtest eingesetzt.

### Beispiel 3: Aktivitätsbestimmung der Transaminase C

Der Reaktionsansatz des Enzymtests enthielt in einem Gesamtvolumen von 1 ml: 0,2 ml 0,25 M Tris/HCl, pH 8, 0,005 ml Transaminase-C-Protein, und 0,1 ml 2,5 mM Pyridoxalphosphat, sowie 0,1 ml 40 mM Ketoisocaproat und 0,1 ml 0,5 M L-Alanin, oder 0,1 ml 40 mM Ketoisovalerat und 0,1 ml 0,5 M L-Alanin, oder 0,1 ml 40 mM Ketomethylvalerat und 0,1 ml 0,5 M L-Alanin, oder 0,1 ml 40 mM Ketoisocaproat und 0,1 ml 0,5 M L-Glutamin, oder 0,1 ml 40 mM Ketoisocaproat und 0,1 ml 0,5 M L-Alanin ohne Transaminase C Protein. Der Enzymtest wurde bei 30 °C in einem Thermocycler 5436 der Firma Eppendorf (Hamburg) durchgeführt. Die Reaktion wurde durch Zugabe des Proteins gestartet. Durch Zugabe von 30 µl eines Stoppreagenzes (6,7% (v/v) Perchlorsäure (70 %ig), 40 % (v/v) Ethanol (95 %ig) in Wasser) zu jeweils 50 µl des Testansatzes wurde der Enzymtest gestoppt. Um die Proben für den Nachweis der gebildeten Aminosäuren über reversed phase HPLC vorzubereiten, wurden 20 µl eines Neutralisierunsgspuffers (20 mM Tris, 2,3 M Di-Kalium-carbonat, pH 8) zugegeben. Der durch die Neutralisierung der Perchlorsäure ausfallende Niederschlag wurde abzentrifugiert (13000 Upm, 10 min) und der Überstand in verschiedenen Verdünnungen für die Quantifizierung mittels HPLC eingesetzt. Diese erfolgte nach automatischer Derivatisierung mit o-Phthaldialdehyd wie beschrieben (Hara et al. 1985, Analytica Chimica Acta 172:167-173). Wie Tabelle 1 zeigt, katalysiert das isolierte Protein die L-Alanin abhängige Aminierung von Ketoisovalerat zu L-Valin.

**TABELLE 1**

| Protein | Amino-donator | Amino-acceptor | Produkt | spez. Aktivität |
|---|---|---|---|---|
| Transaminase C | L-Alanin | Ketoiso-caproat | L-Leucin | 0,9 |
| Transaminase C | L-Alanin | Ketoiso-valerat | L-Valin | 18,2 |
| Transaminase C | L-Alanin | Ketomethyl-valerat | L-Iso-leucin | 3,7 |
| Transaminase C | L-Glutamin | Ketoiso-/valerat | L-Valin | 0,1 |
| Kontrolle | L-Alanin | Ketoiso-valerat | L-Valin | 0,0 |

Die spezifische Aktivität (spez. Aktivität) ist in Micromol Produkt pro Minute und Milligramm Transaminase C Protein angegeben.

### Beispiel 4: Überexpression der Transaminase C

Mit Hilfe der PCR Reaktion wurde ein DNA-Fragment, das das Transaminase-C-Gen enthält amplifiziert. Es wurden die folgenden Primer benutzt:
Trans_c_for:
   5'-CGGGATCCAAGGAGATATAGATATGTCTCTTATGAAGCCAAGCA-3'
Trans_c_rev:
   5'- CGGGATCCCTATTTTTTGATGAATTCTCC - 3'

Die angegebenen Primer wurden durch MWG Biotech synthetisiert, und die PCR Reaktion entsprechend Standard Protokollen durchgeführt (Innis et al. PCR Protocols. A guide to Methods and Applications. 1990. Academic Press). Mit den Primern wurde ein DNA-Fragment von etwa 1,1 kb amplifiziert, das für Transaminase C kodiert. Die Primer enthalten zusätzlich die Schnittstelle des Restriktionsenzyms BamHI.

Das amplifizierte DNA-Fragment von etwa 1,1 kb wurde im 0.8%igen Agarosegel identifiziert und aus dem Gel mit bestehenden Methoden isoliert (QIAquik Gel Extraction Kit, Quiagen, Hilden). Die Ligation des Fragments erfolgte mit dem SureCloning Kit (Amersham, UK) in den Expressionsvektor pEKEx2 (Eikmanns et al. 1991, Gene 102:93-8). Mit dem Ligationsansatz wurde E. coli DH5 transformiert (Grant et al., 1990. Proceedings of the National of Sciences of the United States of America USA, 87:4645-4649). Die Selektion Plasmid enthaltender Stämme erfolgte durch Ausplattieren des Transformationsansatzes auf 25 mg pro Liter Kanamycin enthaltenden LB Platten.

Nach Plasmidisolation wurden erhaltene Plasmide durch Restriktionsverdau und gelelektrophoretische Analyse charakterisiert. Das erhaltene Plasmid wurde als pEKEx2ATC bezeichnet.

Das Plasmid pEKEx2ATC sowie das Ausgangsplasmid pEKEx2 wurde zur Transformation des Stammes13032ΔpanBC auf Kanamycinresistenz benutzt. Der Stamm ist in EP1155139B1 beschrieben, und die Transformationstechnik bei Kirchner et al. J Biotechnol. 2003, 104:287-99.

### Beispiel 5: L-Valinbildung

Der Stamm 13032ΔpanBC pEKEx2ATC, sowie der zur Kontroll-Stamm 13032ΔpanBC pEKEx2 wurde in dem Medium CGIII (Menkel et al. 1989, Appl. Environ. Microbiol. 55:684-8) bei 30°C angezogen. Damit wurde das Medium CGXII mit einer Optischen Dichte von 1 beimpft. Das Medium CG12 enthält pro Liter: 20 g (NH₄)₂SO₄, 5 g Harnstoff, 1 g KH₂PO₄, 1 g K₂HPO₄, 0,25 g Mg₂O_{4*}7 H₂O, 42 g 3-Morpholinopropansulfonsäure, 10 mg CaCl₂, 10 mg FeSO_{4*}7 H₂O, 10 mg MnSO₄* H₂O, 1 mg ZnSO_{4*}7 H₂O, 0,2 mg CuSO₄, 0,02 mg NiCl_{2*}6 H₂O, 0,2 mg Biotin, 40 g Glukose, und 0,03 mg Protokatechusäure. Die Kultur wurde bei 30°C und 170 Umdrehungen pro Minute inkubiert, und nach 48 Stunden wurde die L-Valinakkumulation im Medium mittels HPLC bestimmt. Diese erfolgte mit o-Phthaldialdehyd wie beschrieben (Hara et al. 1985, Analytica Chimica Acta 172:167-173). Die bestimmten L-Valinkonzentrationen sind in Tabelle 2 gezeigt.

**TABELLE 2**

| Stamm | L-Valin |
|---|---|
| 13032ΔpanBC pEKEx2ATC | 11,0 mM |
| 13032ΔpanBC pEKEx2 | 8,1 mM |

## Patentansprüche

1. Verfahren zur fermentativen Herstellung von L-Valin, **dadurch gekennzeichnet,**
**dass** in einem coryneformen Bakterium die Aktivität der Transaminase C erhöht wird.

2. Verfahren nach Anspruch 1,
**dadurch gekennzeichnet,**
**dass** die Verstärkung der Aktivität der Transaminase C durch Erhöhen der Genexpression erfolgt.

3. Verfahren nach Anspruch 2,
**dadurch gekennzeichnet,**
**dass** die Erhöhung der Genexpression durch Einführen eines stärkeren Promotors erfolgt.

4. Verfahren nach Anspruch 3,
**dadurch gekennzeichnet,**
**dass** zur Erhöhung der Genexpression ein tac-Promotor oder ein IPTG-induzierbarer Promotor eingesetzt wird.

5. Verfahren nach einem der Ansprüche 2 bis 4,
**dadurch gekennzeichnet,**
**dass** die Verstärkung der Aktivität der Transaminase durch Erhöhen der Genkopienzahl der für die Transaminase C kodierenden Gene und/oder deren Allele erfolgt.

6. Verfahren nach Anspruch 5,
**dadurch gekennzeichnet,**
**dass** die Erhöhung der Genkopienzahl durch Einbringen von Vektoren erfolgt, welche mindestens ein Gen für die Transaminase C und/oder ein Allel davon beinhalten.

7. Verfahren nach Anspruch 6,
**dadurch gekennzeichnet,**
**dass** als Vektor ein Plasmid eingesetzt wird.

8. Verfahren nach Anspruch 7,
**dadurch gekennzeichnet,**
**dass** als Plasmid mindestens eine Komponente aus der Gruppe bestehend aus pZ1 pEKEx1, pHS2-1, pHM1519, pBL1, pGA1, pCG4, pNG2, pAG1 und pEKEx2 eingesetzt wird.

9. Verfahren nach einem der Ansprüche 5 bis 8,
**dadurch gekennzeichnet,**
**dass** die Erhöhung der Genkopienzahl durch Einbringen von für die Transaminase C kodierenden Genen und/oder deren Allele in das chromosomale Genom erfolgt.

10. Verfahren nach einem der Ansprüche 2 bis 9,
**dadurch gekennzeichnet,**
**dass** die Verstärkung der Genexpression durch Veränderung der Signalstrukturen erfolgt.

11. Verfahren nach Anspruch 10,
**dadurch gekennzeichnet,**
**dass** die Veränderung der Signalstrukturen durch mindestens eine Komponente aus der Gruppe der folgen Maßnahmen erfolgt:
- Veränderung der Repressorgene;
- Veränderung der Aktivatorgene,
- Veränderung der Operatoren,
- Veränderung der Promotoren
- Veränderung der Attenuatoren,
- Veränderung der Ribosomenbindungsstellen,
- Veränderung des Startkodons,
- Veränderung der Terminatoren.

12. Verfahren nach einem der Ansprüche 1 bis 11,
**dadurch gekennzeichnet,**
**dass** die m-RNA - Stabilität erhöht wird.

13. Verfahren nach Anspruch 12,
**dadurch gekennzeichnet,**
**dass** die m-RNA-Stabilität durch Veränderung des 5'-Endes oder des 3'-Endes bewirkt wird.

14. Verfahren nach einem der Ansprüche 1 bis 13,
**dadurch gekennzeichnet,**
**dass** ein Mikroorganismus eingesetzt wird, der in seiner Wildform bereits eine Valinproduktion aufweist.

15. Verfahren nach einem der Ansprüche 1 bis 14,
**dadurch gekennzeichnet,**
**dass** als Mikroorganismus ein Corynebakterium eingesetzt wird.

16. Verfahren nach Anspruch 15,
**dadurch gekennzeichnet,**
**dass** mindestens eine Komponente aus der Gruppe Corynebacterium glutamicum, Corynebacterium acetoglutamicum, Corynebacterium thermoanimogenes, Brevibacterium flavum, Brevibacterium lactofermentum, Brevibacterium divaricatum eingsetzt wird.

17. Verfahren nach Anspruch 16,
**dadurch gekennzeichnet,**
**dass** mindestens eine Komponente aus der Gruppe der Microorganismen
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020 eingesetzt wird.

18. Verfahren nach einem der Ansprüche 1 bis 17,
**dadurch gekennzeichnet,**
**dass** ein Mikroorganismus eingesetzt wird, bei dem mindestens eine Komponente der Gene
- die für die Acetohydroxysäuresynthase kodierenden ilvBN-Gene,
- das für die Isomeroreduktase kodierende ilvC-Gen,
- das für die Dehydratase kodierende ilvD-Gen,
- die für feedback-resistente Acetohydroxysäuresynthase kodierenden ilvBN-Gene,
in ihrer Aktivität verstärkt werden.

19. Verfahren nach einem der Ansprüche 1 bis 18, **dadurch gekennzeichnet, dass**
- die für die Pantothenatsynthese kodierenden panBCD-Gene,
- die für die Liponsäuresynthese kodierenden lipAB-Gene,
- die für die Pyruvatdehydrogenase kodierenden aceE-, aceF, IpD-Gene,
- die für die Gene der ATP synthase A Untereinheit, ATP synthase B Untereinheit, ATP synthase C Untereinheit, ATP synthase alpha Untereinheit, ATP synthase gamma Untereinheit, ATP synthase Untereinheit, ATP synthase epsilon Untereinheit, ATP ynthase delta Untereinheit in ihrer Aktivität inaktiviert oder in ihrer Expression reduziert werden.

20. L-Valin produzierendes Coryneformes Bakterium,
**dadurch gekennzeichnet,**
**dass** es gegenüber seiner Wildform in seiner Transaminase C Aktivität verstärkt ist.

21. Mikroorganismus nach Anspruch 20,
**dadurch gekennzeichnet,**
**dass** seine Genkopienzahl der Transaminase C Gene und/oder der Allele davon erhöht ist

22. Mikroorganismus, nach einem der Ansprüche 20 oder 21,
**dadurch gekennzeichnet,**
**dass** er zur Expression des Transaminase C Gens oder dessen Allele einen tac-Promotor besitzt.

23. Mikroorganismus nach einem der Ansprüche 20 bis 22,
**dadurch gekennzeichnet,**
**dass** er über eine erhöhte endogene Genkopienzahl verfügt.

24. Mikroorganismus nach einem der Ansprüche 20 bis 23,
**dadurch gekennzeichnet,**
**dass** er mit Vektoren transformiert ist, welche mindestens ein Transaminase C Gen und/oder deren Allele enthalten.

25. Mikroorganismus nach einem der Ansprüche 20 bis 23,
**dadurch gekennzeichnet,**
**dass** mindestens eine Komponente aus der Gruppe folgender Signalstrukturen verändert ist, die für die Regulation der Expression der Transaminase C verantwortlich sind:
- Repressorgene,
- Aktivatorgene,
- Operatoren,
- Promotoren
- Attenuatoren,
- Ribosomenbindungsstellen,
- Startkodons,
- Terminatoren.

26. Mikroorganismus nach Anspruch 25,
**dadurch gekennzeichnet,**
**dass** er mindestens eine Komponente aus der Gruppe bestehend aus:
Corynebacterium glutamicum, Corynebacterium acetoglutamicum, Corynebacterium thermoanimogenes, Brevibacterium flavum, Brevibacterium lactofermentum, Brevibacterium divaricatum ist.

27. Mikroorganismus nach Anspruch 25 oder 26,
**dadurch gekennzeichnet,**
**dass** er mindestens eine Komponente aus der Gruppe bestehend aus :
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
ist.

28. Mikroorganismus nach einem der Ansprüche 20 bis 28,
**dadurch gekennzeichnet,**
**dass** mindestens eine Komponente aus der Gruppe der Gene:
- die für die Acetohydroxysäuresynthase kodierenden ilvBN-Gene,
- das für die Isomeroreduktase kodierende ilvC-Gen,
- das für die Dehydratase kodierende ilvD-Gen,
- die für feedback-resistente Acetohydroxysäuresynthase kodierenden ilvBN-Gene,
in ihrer Aktivität verstärkt ist.

29. Mikroorganismus nach einem der Ansprüche 20 bis 28,
**dadurch gekennzeichnet,**
**dass** mindestens eine Komponente aus der Gruppe der Gene:
- die für die Pantothenatsynthese kodierenden panBCD-Gene,
- die für die Liponsäuresynthese kodierenden lipAB-Gene,
- die für die Pyruvatdehydrogenase kodierenden aceE-, aceF, IpD-Gene,
- die für die Gene der ATP synthase A Untereinheit, ATP synthase B Untereinheit, ATP synthase C Untereinheit, ATP synthase alpha Untereinheit, ATP synthase gamma Untereinheit, ATP synthase Untereinheit, ATP synthase epsilon Untereinheit, ATP synthase delta Untereinheit
in ihrer Aktivität inaktiviert oder in ihrer Expression reduziert werden.

## Claims

1. Process for the fermentative production of L-valine,
**characterised in that**
the activity of transaminase C is enhanced in a coryneforme bacterium.

2. Process according to claim 1,
**characterised in that**
the increase in the activity of transaminase C is produced by enhancing the gene expression.

3. Process according to claim 2,
**characterised in that**
the enhancement of the gene expression is produced by introducing a stronger promoter.

4. Process according to claim 3,
**characterised in that**
a tac promoter or an IPTG inducible promoter is used to enhance the gene expression.

5. Process according to one of claims 2 to 4,
**characterised in that**
the increase in the activity of transaminase is produced by enhancing the gene copy number of the gene coding for transaminase C and/or its alleles.

6. Process according to claim 5,
**characterised in that**
the enhancement of the gene copy number is produced by introducing vectors, which contain at least one gene for transaminase C and/or an allele of it.

7. Process according to claim 6,
**characterised in that**
a plasmid is used as the vector.

8. Process according to claim 7,
**characterised in that**
at least one component from the group consisting of pZ1, pEKExl, pHS2 - 1, pHM1519, pBL1, pGA1, pCG4; pNG2, pAG1 and pEKEx2 is used as the plasmid.

9. Process according to claims 5 to 8,
**characterised in that**
the enhancement of the gene copy number is produced by introducing genes coding for transaminase C and/or its alleles into the chromosomal genome.

10. Process according to one of claims 2 to 9,
**characterised in that**
the increase in the gene expression is produced through changing the signal structures.

11. Process according to claim 10,
**characterised in that**
the change in the signal structures is produced through at least one component from the group of the following measures:
- change of repressor genes,
- change of activator genes,
- change of operators,
- change of promoters
- change of attenuators,
- change of ribosome binding sites,
- change of start codon,
- change of terminators.

12. Process according to one of claims 1 to 11,
**characterised in that**
the m-RNA stability is enhanced.

13. Process according to claim 12.
**characterised in that**
the m-RNA stability is produced by changing the 5' end or the 3' end.

14. Process according to one of claims 1 to 13,
**characterised in that**
a microorganism is used, which already has valine production in its wild form.

15. Process according to one of claims 1 to 14,
**characterised in that**
a coryne bacterium is used as the micoorganism.

16. Process according to claim 15,
**characterised in that**
at least one component from the coryne bacterium glutamicum, coryne bacterium acetoglutamicum, coryne bacterium thermoanimogenes, brevibacterium flavum, brevibacterium lactofermentum, brevibacterium divaricatum group is used.

17. Process according to claim 16,
**characterised in that**
at least one component from the group of microorganisms
coryne bacterium, glutamicum ATCC13032
coryne bacterium acetoglutamicum ATCC15806
coryne bacterium acetoacidophilum ATCC13870
coryne bacterium thermoaminogenes FERM BP-1539
brevibacterium flavum ATCC14067
brevibacterium lactofermentum ATCC13869 and
brevibacterium divaricatum ATCC14020
is used.

18. Process according to one of claims 1 to 17,
**characterised in that**
a microorganism is used, in which there is an increase in the activity of at least one component of the genes
- the ilvBN gene coding for acetohydroxyacid synthase
- the ilvC gene coding for isomer reductase,
- the ilvD gene coding for dehydratase,
- the ilvBN gene coding for feedback resistant acetohydroxyacid synthase.

19. Process according to one of claims 1 to 18,
**characterised in that**
the activity has been inactivated of or there has been a reduction in the expression of
- the panBCD gene coding for pantothenate synthetase,
- the lipAB gene coding for lipoic acid synthetase,
- the aceE, aceF, IpD gene coding for pyruvate dehydrogenase,
- the genes for the ATP synthase A subunit, ATP synthase B subunit, ATP synthase C subunit, ATP synthase alpha subunit, ATP synthase gamma subunit, ATP synthase subunit, ATP synthase epsilon subunit, ATP synthase delta subunit.

20. L-valine producing coryneforme bacterium,
**characterised in that**
its transaminase C activity is increased compared with its wild form.

21. Microorganism according to claim 20,
**characterised in that**
its gene copy number of the transaminase C gene and/or its alleles is enhanced.

22. Microorganism according to one of claims 20 or 21,
**characterised in that**
it has a tac promoter for expression of the transaminase C gene or its alleles.

23. Microorganism according to one of claims 20 to 22,
**characterised in that**
it has an enhanced endogenous gene copy number.

24. Microorganism according to one of claims 20 to 23,
**characterised in that**
it is transformed with vectors, which contain at least one transaminase C gene and/or its alleles.

25. Microorganism according to one of claims 20 to 23,
**characterised in that**
at least one component from the group of the following signal structures is changed, which are responsible for regulation of the expression of transaminase C:
- repressor genes,
- activator genes,
- operators,
- promoters,
- attenuators
- ribosome binding sites,
- start codon,
- terminators.

26. Microorganism according to claim 25,
**characterised in that**
it is at least one component from the group consisting of:
coryne bacterium glutamicum, coryne bacterium acetoglutamicum, coryne bacterium themoanimogenes, brevibacterium flavum, brevibacterium lactofermentum, brevibacterium divaricatum.

27. Microorganism according to claim 25 or 26,
**characterised in that**
it is at least one component from the group consisting of:
coryne bacterium glutamicum ATCC13032
coryne bacterium acetoglutamicum ATCC15806
coryne bacterium thermoaminogenes FERM BP-1539
brevibacterium flavum ATCC14067
brevibacterium lactofermentum ATCC13869 and
brevibacterium divaricatum ATCC14020.

28. Microorganism according to one of claims 20 to 28,
**characterised in that**
there is an increase in the activity of at least one component from the group of genes:
- the ilvBN gene coding for acetohydroxyacid synthase,
- the ilvC gene coding for isomer reductase,
- the ilvD gene coding for dehydratase,
- the ilvBN gene coding for feedback resistant acetohydroxyacid synthase.

29. Microorganism according to one of claims 20 to 28,
**characterised in that**
the activity has been inactivated of or there has been a reduction in the expression of at least one component from the group of genes:
- the panBCD gene coding for pantothenate synthetase,
- the lipAB gene coding for lipoic acid synthetase,
- the aceE, aceF, IpD gene coding for pyruvate dehydrogenase,
- the genes for the ATP synthase A subunit, ATP synthase B subunit, ATP synthase C subunit, ATP synthase alpha subunit, ATP synthase gamma subunit, ATP synthase subunit, ATP synthase epsilon subunit, ATP synthase delta subunit.

## Revendications

1. Procédé de production de L-valine par fermentation, **caractérisé en ce que** l'activité de transaminase C est accrue dans une bactérie corynéforme.

2. Procédé selon la revendication 1, **caractérisé en ce que** le renforcement de l'activité de la transaminase C s'effectue par l'augmentation de l'expression génique.

3. Procédé selon la revendication 2, **caractérisé en ce que** l'augmentation de l'expression génique s'effectue par introduction d'un promoteur plus puissant.

4. Procédé selon la revendication 3, **caractérisé en ce que**, pour augmenter l'expression génique, on utilise un promoteur tac ou un promoteur inductible par IPTG.

5. Procédé selon l'une quelconque des revendications 2 à 4, **caractérisé en ce que** le renforcement de l'activité de la transaminase s'effectue par augmentation du nombre de copies des gènes codant pour la transaminase C et/ou leurs allèles.

6. Procédé selon la revendication 5, **caractérisé en ce que** l'augmentation du nombre de copies de gènes s'effectue par insertion de vecteurs qui contiennent au moins un gène de la transaminase C et/ou un allèle de celui-ci.

7. Procédé selon la revendication 6, **caractérisé en ce que** l'on utilise un plasmide comme vecteur.

8. Procédé selon la revendication 7, **caractérisé en ce que** l'on utilise comme plasmide, au moins un composant du groupe comprenant pZ1, pEKEx1, pHS2-1, pHM1519, pBL1, pGA1, pCG4, pNG2, pAG1 et pEKEx2.

9. Procédé selon l'une des revendications 5 à 8, **caractérisé en ce que** l'augmentation du nombre de copies de gènes s'effectue par insertion de gènes codants pour la transaminase C et/ou leurs allèles dans le génome chromosomique.

10. Procédé selon l'une des revendications 2 à 9, **caractérisé en ce que** le renforcement de l'expression génique s'effectue par la modification des structures de signal.

11. Procédé selon la revendication 10, **caractérisé en ce que** la modification des structures de signal s'effectue par au moins un composant du groupe des mesures suivantes :
- modification des gènes répresseurs,
- modification des gènes activateurs,
- modification des opérateurs,
- modification des promoteurs,
- modification des atténuateurs,
- modification des sites de liaison ribosomiques,
- modification du codon de départ,
- modification des terminateurs.

12. Procédé selon l'une des revendications 1 à 11, **caractérisé en ce que** l'ARNm augmente la stabilité.

13. Procédé selon la revendication 12, **caractérisé en ce que** la stabilité de l'ARNm est entraînée par la modification de l'extrémité 5' ou de l'extrémité 3'.

14. Procédé selon l'une des revendications 1 à 13, **caractérisé en ce qu'**un microorganisme est utilisé, présentant déjà sous sa forme sauvage, une production de valine.

15. Procédé selon l'une des revendications 1 à 14, **caractérisé en ce que** l'on utilise comme microorganisme, une corynébactérie.

16. Procédé selon la revendication 15, **caractérisé en ce que** l'on utilise au moins un composant du groupe comprenant Corynebacterium glutamicum, Corynebacterium acetoglutamicum, Corynebacterium thermoanimogenes, Brevibacterium flavum, Brevibacterium lactofermentum, Brevibacterium divaricatum.

17. Procédé selon la revendication 16, **caractérisé en ce que** l'on utilise au moins un composant du groupe de microorganismes comprenant
Corynebacterium glutamicum ATCC13032,
Corynebacterium acetoglutamicum ATCC15806,
Corynebacterium acetoacidophilum ATCC13870,
Corynebacterium thermoaminogenes FERM BP-1539,
Brevibacterium flavum ATCC14067,
Brevibacterium lactofermentum ATCC13869 et
Brevibacterium divaricatum ATCC14020.

18. Procédé selon l'une des revendications 1 à 17, **caractérisé en ce que** l'on utilise un microorganisme dans lequel au moins un composant des gènes :
- gènes ilvBN codant pour l'acide acétohydroxylique,
- gène ilvC codant pour l'isoméroréductase,
- gène ilvD codant pour la déshydratase,
- gènes ilvBN codants pour l'acide acétohydroxylique résistant à une rétroaction,
est renforcé dans son activité.

19. Procédé selon l'une des revendications 1 à 18, **caractérisé en ce que**
- les gènes panBCD codant pour la synthèse du pantothénate,
- les gènes lipAB codant pour la synthèse de l'acide liponique,
- les gènes aceE, aceF, IpD codant pour la pyruvate-désydrogénase,
- la sous-unité pour le gène de l'ATP synthase A, la sous-unité de l'ATP synthase B, la sous-unité de l'ATP synthase C, la sous-unité de l'ATP synthase alpha, la sous-unité de l'ATP synthase gamma, la sous-unité de l'ATP synthase, la sous-unité de l'ATP synthase epsilon, la sous-unité de l'ATP synthase delta sont inactivés dans leur activité ou leur expression est réduite.

20. Bactérie corynéforme produisant de la L-valine, **caractérisée en ce que,** par rapport à sa forme sauvage, elle est renforcée au niveau de son activité de transaminase C.

21. Microorganisme selon la revendication 20, **caractérisé en ce que** son nombre de copies de gènes codant pour la transaminase C et/ou de ses allèles est accru.

22. Microorganisme selon l'une des revendications 20 ou 21, **caractérisé en ce qu'**il possède, pour l'expression du gène de la transaminase C ou ses allèles, un promoteur tac.

23. Microorganisme selon l'une des revendications 20 à 22, **caractérisé en ce qu'**il dispose d'un nombre de copies de gènes endogènes accru.

24. Microorganisme selon l'une des revendications 20 à 23, **caractérisé en ce qu'**il est transformé par des vecteurs qui contiennent au moins un gène de la transminase C et/ou ses allèles.

25. Microorganisme selon l'une des revendications 20 à 23, **caractérisé en ce qu'**au moins un composant du groupe des structures de signal suivantes, qui est responsable de la régulation de l'expression de la transaminase C, est modifie :
- gènes répresseurs,
- gènes activateurs,
- opérateurs,
- promoteurs,
- atténuateurs,
- sites de liaison ribosomiques,
- codon de départ,
- terminateurs.

26. Microorganisme selon la revendication 25, **caractérisé en ce qu'**il est au moins un composant du groupe comprenant : Corynebacterium glutamicum, Corynebacterium acetoglutamicum, Corynebacterium thermoanimogenes, Brevibacterium flavum, Brevibacterium lactofermentum, Brevibacterium divaricatum.

27. Microorganisme selon la revendication 25 ou 26, **caractérisé en ce qu'**il est au moins un composant du groupe comprenant :
Corynebacterium glutamicum ATCC13032,
Corynebacterium acetoglutamicum ATCC15806,
Corynebacterium acetoacidophilum ATCC13870,
Corynebacterium thermoaminogenes FERM BP-1539,
Brevibacterium flavum ATCC14067,
Brevibacterium lactofermentum ATCC13869 et
Brevibacterium divaricatum ATCC14020.

28. Microorganisme selon l'une des revendications 20 à 27, **caractérisé en ce qu'**au moins un composant du groupe des gènes :
- gènes ilvBN codant pour l'acide acétohydroxylique,
- gène ilvC codant pour l'isoméroréductase,
- gène ilvD codant pour la déshydratase,
- gènes ilvBN codants pour l'acide acétohydroxylique résistant à une rétroaction,
est renforcé dans son activité.

29. Microorganisme selon l'une des revendications 20 à 28, **caractérisé en ce que** au moins un composant du groupe de gènes comprenant :
- les gènes panBCD codant pour la synthèse du pantothénate,
- les gènes lipAB codant pour la synthèse de l'acide liponique,
- les gènes aceE, aceF, IpD codant pour la pyruvate-désydrogénase,
- la sous-unité pour le gène de l'ATP synthase A, la sous-unité de l'ATP synthase B, la sous-unité de l'ATP synthase C, la sous-unité de l'ATP synthase alpha, la sous-unité de l'ATP synthase gamma, la sous-unité de l'ATP synthase, la sous-unité de l'ATP synthase epsilon, la sous-unité de l'ATP synthase delta, sont inactivés dans leur activité ou leur expression est réduite.
